# EUROPEAN PATENT APPLICATION

(11) **EP 2 883 547 A1**
(43) Date of publication of application: **17.06.2015**
(21) Application number: 12882840.7
(22) Date of filing: 08.08.2012
(51) Int. Cl.: A61K 31/47, A61K 9/20, A61K 47/32, A61K 47/38, A61P 3/06

(54) **MEDICINE**

(71) Applicant: Kowa Company, Ltd., Nagoya-shi, Aichi-ken 460-8625 (JP)
(72) Inventor: NISHIDA, Chisa, Fuji-shi, Shizuoka 417-8650 (JP)
(74) Representative: Blodig, Wolfgang
(86) International application number: PCT/JP2012/070163
(87) International publication number: WO 2014/024268

(57) **Abstract**

The present invention provides a pharmaceutical product which includes a solid preparation comprising pitavastatin or a salt thereof, in which production of a lactone form thereof is suppressed.

The pharmaceutical product is characterized by including a solid preparation comprising the following ingredients (A) and (B): (A) pitavastatin or a salt thereof; and (B) at least one member selected from the group consisting of carmellose and a salt thereof, crospovidone, and microcrystalline cellulose, and the solid preparation having a water content of 2.9 mass% or less, wherein the solid preparation is stored in a tight package.

## Description

### Technical Field

The present invention relates to a solid preparation comprising pitavastatin or a salt thereof in which production of a lactone form of pitavastatin is suppressed, and to a pharmaceutical product employing the solid preparation.

### Background Art

Pitavastatin or a salt thereof; e.g., pitavastatin calcium (chemical name: (+)-monocalcium bis{(3R,5S,6E)-7-[2-cyclopropyl-4-(4-fluorophenyl)-3-quinolyl]-3,5-dihydroxy-6-heptenoate}), is a statin compound which is known to have excellent HMG-CoA reductase inhibitory activity and to serve as a useful active ingredient of a therapeutic agent for hyperlipemia, hypercholesterolemia, or the like (Patent Document 1).

Such statin compounds have a dihydroxycarboxylic acid skeleton as a common skeleton, and the skeleton is known to cyclize in the statin molecule through dehydration condensation, to thereby produce a lactone form thereof which has low HMG-CoA reductase inhibitory activity. In pharmaceutical preparations, production of a lactone form may cause a drop in pharmaceutical efficacy and non-uniformity in efficacy between pharmaceutical products. Therefore, various attempts have been made to enhance stability of statin compounds, including pitavastatin contained in pharmaceutical preparations. Most of the attempts are focused on behavior of statin compounds under varied pH conditions. One specific attempt is incorporation of a basic substance such as calcium carbonate into a pharmaceutical preparation so as to maintain a statin compound under basic pH conditions, in consideration of low stability of a dihydroxycarboxylic acid skeleton under low pH conditions (Patent Documents 2 and 3).

Patent Document 4 discloses that when crystal form A of pitavastatin calcium is dried, an amorphous form thereof is formed at a water content of 4% or lower to decrease crystallinity, and that the formed amorphous pitavastatin calcium has considerably poor storage stability. Non-Patent Document 1 discloses, in the section "Stability of active ingredients under various conditions," that the water content decreased and more analogous substances formed under low moisture conditions (60°C, 30% RH).

### Citation list

### Patent Documents

Patent Document 1: JP-A-1989-279866
Patent Document 2: JP-B-2774037
Patent Document 3: JP-B-3276962
Patent Document 4: Japanese Unexamined Patent Publication No. 2007-516952
Patent Document 5: WO2007/52592

### Non-Patent Documents

Non-Patent Document 1: "LIVALO Tablet 1 mg, LIVALO Tablet 2 mg, LIVALO Tablet 4 mg" Interview Form, June, 2012

### Summary of the Invention

### Problems to be Solved by the Invention

However, neither Patent Document 4 nor Non-Patent Document 1 discloses instability of a solid preparation containing pitavastatin calcium and a specific disintegrant having a high hygroscopicity, with moisture absorption (i.e., an increase in water content).

Meanwhile, pharmaceutically acceptable additives such as carmellose or a salt thereof such as carmellose calcium or croscarmellose sodium; crospovidone; and microcrystalline cellulose have considerably high hygroscopicity. By virtue of high hygroscopicity, such additives incorporated into a solid preparation absorb water and swell, or introduce water into the preparation via capillary action. As a result, the solid preparation can be provided with a favorable disintegration property. Therefore, such additives are employed as a disintegrant or the like.

Enhancement in disintegration property of a solid preparation is advantageous in that release of an active ingredient and attainment of a pharmaceutical effect are ensured, to thereby enhance quality of the solid preparation. An orally disintegrating solid preparation, having a favorable disintegration property, can be taken without water. Thus, such an oral solid preparation can be taken at any time, place, and occasion, resulting in improving compliance.

In consideration of such advantages, the present inventor mixed pitavastatin or a salt thereof with a disintegrant such as carmellose or a salt thereof, crospovidone, or microcrystalline cellulose, in order to produce a solid preparation which comprises pitavastatin or a salt thereof and which has a favorable disintegration property, resulting in finding that production of a lactone form increased over time.

Thus, the present invention relates to provide a solid preparation comprising pitavastatin or a salt thereof, and at least one member selected from the group consisting of carmellose and a salt thereof, crospovidone, and microcrystalline cellulose, in which production of a lactone form of pitavastatin is suppressed, and also to provide a pharmaceutical product employing the solid preparation.

### Means for Solving the Problems

In order to solve the aforementioned problems, the present inventor investigated the cause and mechanism of production of a lactone form during mixing pitavastatin or a salt thereof with at least one member selected from the group consisting of carmellose and a salt thereof, crospovidone, and microcrystalline cellulose. As a result, quite surprisingly, it was found that the amount of lactone form which is a dehydration condensation product was correlated with a water content of the mixture and increased with increased amount of water content. Meanwhile, in "stability of drug preparations under various conditions" disclosed in Patent Document 5, stability of LIVALO Tablet products currently available on the market is evaluated through a stress testing (humidity: 60% RH or 85% RH, at 25°C) only as "within the standard" or "decrease in dissolution rate." Thus, the cause and mechanism of production of a lactone form in the co-presence of the aforementioned disintegrant has been suggested to be attributed to high hygroscopicity of the aforementioned specific disintegrant which is not contained in LIVALO Tablet products currently available on the market. The inventor also found that production of a lactone form originating from pitavastatin or a salt thereof can be suppressed through adjusting the water content of the solid preparation to a certain level or lower. The present invention has been accomplished on the basis of these findings.

Accordingly, the present invention provides a pharmaceutical product comprising a solid preparation comprising the following ingredients (A) and (B):
(A) pitavastatin or a salt thereof; and
(B) at least one member selected from the group consisting of carmellose and a salt thereof, crospovidone, and microcrystalline cellulose,
   and the solid preparation having a water content of 2.9 mass% or less, wherein the solid preparation is stored in a tight package.

The present invention also provides a solid preparation comprising the following ingredients (A) and (B):
(A) pitavastatin or a salt thereof; and
(B) at least one member selected from the group consisting of carmellose and a salt thereof, crospovidone,
   and microcrystalline cellulose,
   and the solid preparation having a water content of 2.9 mass% or less.

### Effects of the Invention

In the solid preparation of the present invention, production of a lactone form originating from pitavastatin or a salt thereof is suppressed. In addition, the solid preparation has an excellent disintegration property. That is, the solid preparation of the present invention can maintain the stability of an active ingredient and can ensure release of the active ingredient and attainment of a pharmaceutical effect, to thereby enhance quality of the solid preparation.

In the pharmaceutical product of the present invention, the solid preparation of the present invention is packed by means of a tight protective package. Thus, entry of water into the package is prevented, and the water content of the solid preparation accommodated in the package can be reliably maintained for a long period of time. Thus, production of a lactone form originating from pitavastatin or a salt thereof is suppressed in the solid preparation for a long period of time. Therefore, the pharmaceutical product of the present invention is useful.

### Modes for Carrying Out the Invention

In the present invention, the term "pitavastatin or a salt thereof" (hereinafter may also be referred to as "ingredient (A)") refers to pitavastatin and pharmaceutically acceptable salts of pitavastatin (e.g., alkali metal (e.g., sodium and potassium) salts; alkaline earth metal (e.g., calcium and magnesium) salts; organic amine salts such as phenethylamine salt, and ammonium salts); and further encompasses solvates of pitavastatin or a pharmaceutically acceptable salt with water, alcohol, or the like. In the present invention, one or two or more members thereof may be used in combination.

In the present invention, pitavastatin calcium (chemical name: (+)-monocalcium bis{(3R,5S,6E)-7-[2-cyclopropyl-4-(4-fluorophenyl)-3-quinolyl]-3,5-dihydroxy-6-heptenoate}) is preferred.

Pitavastatin and salts thereof are known compounds. They may be produced through a method disclosed in, for example, JP-A-1989-279866 or US Patent No. 5856336.

In the present invention, no particular limitation is imposed on the ingredient (A) content of the solid preparation, and may be appropriately adjusted depending on the sex, age, condition, etc. of the subject. In one preferred embodiment of the solid preparation, the ingredient (A) content, as reduced to pitavastatin calcium, is 0.1 to 16 mg in a dosage unit, more preferably 0.5 to 12 mg, even more preferably 1 to 8 mg, particularly preferably 1 to 4 mg.

The solid preparation of the present invention preferably has an ingredient (A) content, based on the total mass of the solid preparation and as reduced to pitavastatin calcium, of 0.1 to 10 mass%, more preferably 0.2 to 5 mass%, particularly preferably 0.5 to 4 mass%.

As used herein, the expression "at least one member selected from the group consisting of carmellose and a salt thereof, crospovidone, and microcrystalline cellulose" (hereinafter may be also be referred to "ingredient (B)") encompasses carmellose, a pharmaceutically acceptable salt of carmellose, a cross-linked polymer of carmellose or a pharmaceutically acceptable salt of carmellose, crospovidone, and microcrystalline cellulose. In the present invention, these members may be used singly or in combination of two or more species.

In the present invention, no particular limitation is imposed on the ingredient (B) content of the solid preparation, and it may be appropriately adjusted in accordance with the disintegration property of the solid preparation. The solid preparation of the present invention preferably has a total ingredient (B) content, based on the total mass of the solid preparation, of 0.1 to 85 mass%, more preferably 0.5 to 70 mass%, particularly preferably 1 to 60 mass%. The total ingredient (B) content of the solid preparation, with respect to 1 part by mass (as reduced to pitavastatin calcium) of ingredient (A), is preferably 0.1 to 90 parts by mass, more preferably 0.5 to 75 parts by mass, particularly preferably 1 to 65 parts by mass.

In the present invention, the term "carmellose and a salt thereof" refers to carmellose and pharmaceutically acceptable salts of carmellose (e.g., alkali metal (e.g., sodium and potassium) salts; and alkaline earth metal (e.g., calcium) salts), and further encompasses a cross-linked polymer of carmellose or a pharmaceutically acceptable salt of carmellose (i.e., croscarmellose). Specific examples include carmellose, carmellose potassium, carmellose calcium, carmellose sodium, and croscarmellose sodium. In the present invention, these members may be used singly or in combination of two or more species.

In the present invention, carmellose and a salt thereof having any different molecular weight or salt species may be employed. Among them, carmellose calcium, carmellose sodium, and croscarmellose sodium are preferred, with carmellose calcium and carmellose sodium being particularly preferred.

Carmellose and a salt thereof used in the present invention may be commercial products. Specific examples include NS-300 and ECG-505 (products of Gotoku Chemical Company Ltd.), Sunrose (product of Nippon Paper Chemicals Co., Ltd.), Cellogen (product of San-Ei Gen F.F.I. Inc.), Cellogen series (product of Dai-Ichi Kogyo Seiyaku Co., Ltd.), Ac-Di-Sol (product of Asahi Kasei Chemicals Corporation), and Kiccolate ND-2HS (product of Nichirin Chemical Industries, Ltd.).

In the present invention, no particular limitation is imposed on the total amount of carmellose and a salt thereof in the solid preparation, and it may be appropriately adjusted in accordance with the disintegration property of the solid preparation. The solid preparation of the present invention preferably has a total amount of carmellose and a salt thereof, based on the total mass of the solid preparation, of 0.01 to 25 mass%, more preferably 0.1 to 20 mass%, particularly preferably 0.5 to 15 mass%. The total amount of carmellose and a salt thereof in the solid preparation, with respect to 1 part by mass (as reduced to pitavastatin calcium) of ingredient (A), is preferably 0.01 to 30 parts by mass, more preferably 0.1 to 25 parts by mass, particularly preferably 0.5 to 20 parts by mass.

No particular limitation is imposed on the crospovidone employed in the present invention, and crospovidone having any different molecular weight or the like may be used. Such crospovidone species may be used singly or in combination of two or more species.

Crospovidone species used in the present invention may be commercial products. Specific examples include crospovidone (product of Gokyo Sangyo), Kollidon CL-F, Kollidon CL-M, and Kollidon CL-SF (products of BASF), and Polyplasdone XL, Polyplasdone XL-10, and Polyplasdone INF-10 (products of ISP Japan).

In the present invention, no particular limitation is imposed on the crospovidone content of the solid preparation, and it may be appropriately adjusted in accordance with the disintegration property of the solid preparation. The solid preparation of the present invention preferably has a crospovidone content, based on the total mass of the solid preparation, of 0.1 to 25 mass%, more preferably 0.5 to 20 mass%, particularly preferably 1 to 15 mass%. The crospovidone content of the solid preparation, with respect to 1 part by mass (as reduced to pitavastatin calcium) of ingredient (A), is preferably 0.1 to 30 parts by mass, more preferably 0.5 to 25 parts by mass, particularly preferably 1 to 20 parts by mass.

No particular limitation is imposed on the microcrystalline cellulose employed in the present invention, and microcrystalline cellulose having any different molecular weight, particle size, or the like may be used. Such microcrystalline cellulose species may be used singly or in combination of two or more species.

Microcrystalline cellulose species used in the present invention may be commercial products. Specific examples include Ceolus UF grade, Ceolus KG grade, and Ceolus PH grade (products of Asahi Kasei Chemicals Corporation).

In the present invention, no particular limitation is imposed on the microcrystalline cellulose content of the solid preparation, and it may be appropriately adjusted in accordance with the disintegration property of the solid preparation. The solid preparation of the present invention preferably has a microcrystalline cellulose content, based on the total mass of the solid preparation, of 0.1 to 80 mass%, more preferably 0.5 to 65 mass%, particularly preferably 1 to 50 mass%. The microcrystalline cellulose content of the solid preparation, with respect to 1 part by mass (as reduced to pitavastatin calcium) of ingredient (A), is preferably 0.1 to 85 parts by mass, more preferably 0.5 to 70 parts by mass, particularly preferably 1 to 55 parts by mass.

The solid preparation of the present invention is required to have a water content of 2.9 mass% or less, based on the total mass of the solid preparation, for suppressing production of a lactone form. From the viewpoint of suppression of lactone form production, the water content is more preferably 2.1 mass% or less, particularly preferably 1.9 mass% or less.

As described in Test Example 3 given hereinbelow, when the water content of the solid preparation of the present invention is adjusted to 1.5 mass% or more, production of a 5-keto form, which is another decomposition product differing from the lactone form, can be suppressed. As disclosed in Patent Document 5, the 5-keto form is known to be a photo-decomposition product of pitavastatin or a salt thereof. However, there has been no report about correlation between production of the 5-keto form and water content.

In the solid preparation of the present invention having a water content of 1.5 to 2.9 mass% (preferably 1.5 to 2.1 mass%, particularly preferably 1.5 to 1.9 mass%), production of a lactone form and the 5-keto form is suppressed. Thus, stability of pitavastatin in the solid preparation is remarkably high, which is a highly advantageous effect of the present invention.

In another aspect of the present invention, there is provided a solid preparation comprising pitavastatin or a salt thereof and having a water content of 1.5 mass% or more, preferably a solid preparation comprising the following ingredients (A) and (B):
(A) pitavastatin or a salt thereof; and
(B) at least one member selected from the group consisting of carmellose and a salt thereof, crospovidone, and microcrystalline cellulose,
   and the solid preparation having a water content of 1.5 mass% or more.

The water content of the solid preparation of the present invention is determined through the Karl Fischer method. Specifically, the water content may be determined in accordance with "Water Determination (Karl Fischer Method)" described in "The Japanese Pharmacopoeia (16th)." The specific technique of the method may be appropriately selected from volumetric titration and amperometric titration.

One characteristic feature of the present invention resides in that the water content of the solid preparation comprising ingredients (A) and (B) is adjusted, to thereby suppress production of a lactone form originating from ingredient (A). Examples of the means for adjusting the water content of the solid preparation include humidifying means and drying means. The water content may be adjusted to a target level of the present invention through combination of the two means.

In one example of the humidifying means, a water-containing solvent is used as a kneading liquid in wet granulation.

Examples of the drying means include a drying apparatus and a drying agent. The drying apparatus may be such an apparatus as generally employed in the fields of pharmaceuticals and food. Specific examples include a box-type drier, a fluidized-bed drier, a spray drier, a freeze drier, a vacuum drier, and a high-frequency drier. The drying agent may be such an agent as generally employed in the fields of pharmaceuticals and food. Specific examples include one or more members selected from the group consisting of silica gel, silica alumina gel (e.g., allophane), natural zeolite, synthetic zeolite (e.g., molecular sieve), quick lime (calcium oxide), bentonite clay (e.g., montmorillonite), calcium chloride, magnesium chloride, and magnesium oxide. These drying agents may be blended with activated carbon. In the present invention, a technique employing a drying apparatus is preferred, from the viewpoint of ease of adjusting the water content of the solid preparation.

The aforementioned humidifying means or drying means may be employed during or after production of the solid preparation. However, in order to correctly adjust the water content of the solid preparation, the humidifying means or drying means is preferably employed at least after production of the solid preparation.

In addition to the aforementioned ingredients, the solid preparation of the present invention may further comprise additives generally employed in the art, in accordance with the specific form (dosage form) thereof. Examples of such additives include a diluent, a disintegrant (except for ingredient (B)), a binder, a lubricant, a colorant, a plasticizer, a film-forming agent, a water-insoluble polymer, an antioxidant, a corrigent, a sweetener, a pH-adjusting agent, a surfactant, a perfume, or the like. These additives may be used singly or in combination of two or more species. The amount(s) of the additive(s) may be appropriately set.

Examples of the diluent include inorganic diluents such as titanium oxide, aluminum silicate, silicon dioxide, anhydrous sodium sulfate, anhydrous dibasic calcium phosphate, sodium chloride, amorphous silicon oxide hydrate, calcium silicate, light anhydrous silicic acid, heavy anhydrous silicic acid, calcium sulfate, calcium monohydrogen phosphate, dibasic calcium phosphate, dibasic sodium phosphate, monobasic potassium phosphate, monobasic calcium phosphate, monobasic sodium phosphate, and magnesium oxide; and organic diluents such as dextrin powder, starch (wheat starch, rice starch, corn starch, or partially pregelatinized starch), fructose, caramel, agar, xylitol, paraffin, sucrose, fructose, maltose, lactose, white soft sugar, glucose, pullulan, polyoxyethylene hydrogenated castor oil, maltitol, hydrogenated maltose starch syrup, powdered hydrogenated maltose starch syrup, erythritol, xylitol, sorbitol, mannitol, lactitol, trehalose, hydrogenated palatinose, maltose, aminoalkyl methacrylate copolymer E, polyvinyl acetal diethylaminoacetate, calcium citrate, or the like. These diluents may be used singly or in combination of two or more species.

Examples of the disintegrant include starch, sucrose fatty acid ester, gelatin, sodium hydrogencarbonate, dextrin, dehydroacetic acid and a salt thereof, polyoxyethylene hydrogenated castor oil 60, or the like. These disintegrants may be used singly or in combination of two or more species.

Examples of the binder include methylcellulose, hydroxypropylcellulose, hypromellose, starch (wheat starch, rice starch, corn starch, or partially pregelatinized starch), dextrin, pullulan, acacia, agar, gelatin, tragacanth, sodium alginate, polyvinyl alcohol, aminoalkyl methacrylate copolymer E, polyvinyl acetal diethylaminoacetate, or the like. These binders may be used singly or in combination of two or more species.

Examples of the lubricant include calcium stearate, magnesium stearate, sodium stearyl fumarate, sucrose fatty acid ester, or the like. These lubricants may be used singly or in combination of two or more species.

Examples of the colorant include yellow ferric oxide, brown iron oxide, caramel, black iron oxide, titanium oxide, red ferric oxide, tar dye, aluminum lake dye, sodium copper chlorophyllin, or the like. These colorants may be used singly or in combination of two or more species.

Examples of the plasticizer include glycerin, sesame oil, sorbitol, castor oil, propylene glycol, polyoxyethylene polyoxypropylene glycol, Polysorbate 80 (polyoxyethylene(20) sorbitan oleate), polyethylene glycols [e.g., Macrogol 400 (polymerization degree "n" of oxyethylene units: 7 to 9, hereinafter "n" refers to polymerization degree), Macrogol 600 (n: 11 to 13), Macrogol 1500 (equiamount mixture of n: 5 to 6 and n: 28 to 36), Macrogol 4000 (n: 59 to 84), and Macrogol 6000 (n: 165 to 210)], or the like. These plasticizers may be used singly or in combination of two or more species.

Examples of the film-forming agent include alkylcelluloses such as methylcellulose and ethylcellulose; alginic acid and a salt thereof such as sodium alginate; carrageenan; xanthan gum; hydroxyalkylcelluloses such as hydroxymethylcellulose, hydroxyethylcellulose, hydroxypropylcellulose, and hypromellose (hydroxypropylmethylcellulose); hydroxyalkylcellulose phthalates such as hydroxypropylmethylcellulose phthalate; pullulan; polyvinyl acetate; polyvinyl acetate phthalate; polyvinyl alcohol, or the like. These film-forming agents may be used singly or in combination of two or more species.

Examples of the water-insoluble polymer include carboxyvinyl polymer, aminoalkyl methacrylate copolymer, or the like. These water-insoluble polymers may be used singly or in combination of two or more species.

Examples of the antioxidant include ascorbic acid, sodium bisulfite, sodium sulfite, sodium edetate, erythorbic acid, tocopheryl acetate, dibutylhydroxytoluene, natural vitamin E, tocopherol, butylhydroxyanisole, or the like. These antioxidants may be used singly or in combination of two or more species.

Examples of the corrigent include terpenes such as limonene, pinene, camphene, cymene, cineol, citronellol, geraniol, nerol, linalool, menthol, terpineol, rhodinol, borneol, isoborneol, menthone, camphor, eugenol, and cinnzeylanol; terpene-containing essential oils such as bitter orange oil, sweet orange oil, peppermint oil, camphor oil, eucalyptus oil, turpentine oil, lemon oil, ginger oil, clove oil, cassia oil, lavender oil, fennel oil, chamomile oil, perilla oil, and spearmint oil (hereinafter, terpene and terpene-containing essential oils are collectively referred to as "a terpene compound"); and acidulants such as ascorbic acid, tartaric acid, citric acid, malic acid, salts thereof, or the like. These corrigents may be used singly or in combination of two or more species. Of these, a terpene compound is preferred, with menthol being more preferred and 1-menthol being particularly preferred.

Examples of the sweetener include aspartame, stevia, sucralose, glycyrrhizic acid, thaumatin, acesulfame potassium, saccharin, saccharin sodium, or the like. These sweeteners may be used singly or in combination of two or more species. Of these, sucralose is preferred.

No particular limitation is imposed on the pH of the solid preparation of the present invention, and the pH is 4 or higher, preferably 4 to 13, more preferably 5 to 12, even more preferably 6 to 11, particularly preferably 7 to 11. As used herein, the pH of the solid preparation refers to a pH of a liquid obtained by dissolving or dispersing one dosage unit of the solid preparation in purified water (4 mL) in accordance with the pH measurement method described in "The Japanese Pharmacopoeia (16th edition)."

The solid preparation of the present invention may be formed into various dosage forms through a known method described in, for example, "General Rules for Preparations" of "The Japanese Pharmacopoeia (16th edition)." No particular limitation is imposed on the dosage form, and dosage forms described in "General Rules for Preparations" of "The Japanese Pharmacopoeia (16th edition)" may be provided. Specific examples include solid preparations for oral administration such as tablets (including orally disintegrating tablet, chewable tablet, effervescent tablet, dispersible tablet, soluble tablet, and the like), capsules, pills, granules, fine granules, and powders; tablets for oro-mucosal application (including troche, sublingual tablet, buccal tablet, mucoadhesive tablet, and medicated chewing gum); and solid preparations for parenteral application such as suppository, virginal tablet, and virginal suppository. Among them, solid preparations for oral administration are preferred. If necessary, these solid preparations may be coated with film, a sugar coating material, or the like.

The dosage form of the solid preparation of the present invention is preferably tablet, capsule, pill, granule, fine granule, or powder. Among them, tablet is preferred, with orally disintegrating tablet being particularly preferred. When a solid preparation in the form of orally disintegrating tablet is administered, the tablet is rapidly disintegrated in the mouth, thereby facilitating intake. Furthermore, compliance can be enhanced. Thus, advantageous effects can be attained.

In the present invention, no particular limitation is imposed on the disintegration time of the orally disintegrating tablet in the mouth (i.e., the time required for complete disintegration of a solid preparation by saliva in the mouth of a health subject), and the disintegration time varies depending on the dosage form, size, etc. of the solid preparation. The disintegration time is, for example, 90 seconds or shorter, preferably 60 seconds or shorter, particularly preferably 30 seconds or shorter.

Various dosage forms of the solid preparation of the present invention may be produced through known techniques disclosed in "The Japanese Pharmacopoeia (16th edition)" or the like.

Specifically, an orally disintegrating tablet (hereinafter referred to as an "OD tablet"), which is a dosage form preferred in the present invention, may be produced through a technique in which ingredient (A), ingredient (B), and if needed, optional additives or the like are directly compacted; or through a technique including mixing ingredient (A), ingredient (B), and if needed, optional additives or the like, compacting the mixture to plates or slug tablets, pulverizing the tablets, if needed, adding optional additives or the like, and compacting the resultant mixture in a dry state. An alternative production technique includes mixing ingredient (A), ingredient (B), and if needed, optional additives or the like, granulating the mixture through an appropriate method, and compacting the resultant mixture in a dry state.

The OD tablet of the present invention may be produced through filling a mold such as a blister pocket with a suspension or solution containing ingredients (A) and (B), lyophilizing the suspension or solution, and drying the lyophilized product to solid. Notably, the suspension or solution may further contain gelatin, dextran, alginic acid or a salt thereof, sugar alcohol (e.g., erythritol, xylitol, sorbitol, mannitol, or the like), glycine, or the like.

Alternatively, the OD tablet of the present invention may be produced through a so-called wet pelletizing technique in which a mixture containing ingredients (A) and (B) and at least one member selected from saccharides and sugar alcohols is moisturized with water, aqueous alcohol, or the like, and pelletizing the mixture at low pressure.

Yet alternatively, the OD tablet of the present invention may be produced through a technique in which a corresponding mixture is granulated with a sugar having low moldability (e.g., lactose hydrate, mannitol, glucose hydrate, white soft sugar, xylitol, or the like) and a sugar having high moldability (e.g., maltose, maltitol, sorbitol, oligosaccharides such as lactose and fructose, or the like ), and the granulated product is pelletized. In this alternative technique, ingredient (A) or (B) may be present in the granulated product, or may be added to the granulated product after production thereof. Preferably, ingredient (A) or (B) may be present in the granulated product.

Still alternatively, the OD tablet of the present invention may be produced through a technique employing a relatively low-melting-point sugar having a melting point or a decomposition temperature lower than that of ingredient (A) or other additives; e.g., xylitol, trehalose, maltose, sorbitol, erythritol, glucose, maltitol, mannitol, white soft sugar, lactose hydrate, or the like. In a specific procedure, ingredients (A) and (B) and if needed, an optional additive are melted and then solidified, and the solidified product is pelletized. In this alternative technique, ingredient (A) or (B) may be present in the melt-solidified product, or may be added to the melt-solidified product after production thereof. Preferably, ingredient (A) or (B) may be present in the melt-solidified product.

The present invention also provides a pharmaceutical product containing the aforementioned solid preparation which is stored in a tight package. Through storing the solid preparation in a tight package, entry of water from the outside of the package can be prevented, whereby the water content of the solid preparation stored in the package can be constantly maintained for a long period of time. As a result, production of a lactone form originating ingredient (A) in the solid preparation can be suppressed for a long period of time.

A characteristic feature of the "pharmaceutical product" of the present invention resides in that the solid preparation has a water content of 2.9 mass% or less in the tight package. In other words, a water content of 2.9 mass% or less of the solid preparation is ensured, immediately after removal thereof from the tight package. For example, even when the solid preparation has a water content of 2.9 mass% or more before storage in the tight package, the "pharmaceutical product" falls within the scope of the present invention, so long as the water content of the solid preparation is adjusted to 2.9 mass% or less in the tight package through, for example, the co-presence of a desiccant in the package (i.e., so long as the water content of the solid preparation is adjusted to 2.9 mass% or less immediately after removal thereof from the tight package).

In the present invention, the term "tight package" refers to a package that can prevent substantial entry of water thereinto under ordinary conditions of handling, transfer, storage, etc. and is a concept encompassing "tight container" and "hermetic container" defined by "General Rules" of "The Japanese Pharmacopoeia (16th edition)." Any of fixed-form packages and non-fixed-form packages may be used in the present invention. Specific examples thereof include a bottle package, an SP (strip package), a PTP (press through package), a pillow package, a stick package, or the like. In the present invention, these packages may be used in combination of a plurality of members. In one mode thereof, a solid preparation is packed with a PTP and then further with a pillow package.

No particular limitation is imposed on the package material (material) of the tight package, so long as it exhibits moisture resistance, and there may be appropriately used any material that is employed in pharmaceutical and food fields for protecting a content readily damaged by water, or for other purposes.

Examples of the material of the bottle of a bottle package include glass, plastics (e.g., polyester, polyethylene (including low-density polyethylene (LDPE) and high-density polyethylene (HDPE)), polycarbonate, polystyrene, polypropylene, or the like), and metals (e.g., aluminum, or the like), or the like. Examples of the material of the stopper or lid thereof include plastics (e.g., polyester, polyethylene, polycarbonate, polystyrene, polypropylene, or the like), metals (e.g., aluminum, or the like), or the like. In one mode of packing with a bottle package, appropriate pieces of the solid preparation of the present invention are placed in a commercial bottle, and the bottle is sealed with an appropriate stopper or lid. The size of the bottle may be appropriately selected, so long as the size is adapted to the number of solid preparation pieces stored therein. The capacity of the bottle is, for example, about 10 to about 500 mL, preferably 14 to 400 mL, more preferably 24 to 350 mL. The material of the bottle package is preferably polyethylene or polypropylene, more preferably low-density polyethylene (LDPE) or high-density polyethylene (HDPE), particularly preferably high-density polyethylene (HDPE).

Examples of the package material of the SP, PTP, pillow package, stick package, and the like include resins such as biaxially oriented polypropylene (OPP), biaxially oriented polyester (PET), glucose-modified PET (PET-G), biaxially oriented Nylon (ONy, PA), cellophane, paper, low-density polyethylene (LDPE), linear low-density polyethylene (L-LDPE), ethylene-vinyl acetate copolymer (EVA), non-oriented polypropylene (CPP, IPP), ionomer resin (IO), ethylene-methacrylic acid copolymer (EMAA), polyacrylonitrile (PAN), biaxially oriented poly(vinylidene chloride) (PVDC), ethylene-vinyl alcohol copolymer resin (EVOH), poly(vinyl chloride) (PVC), cyclic polyolefin (COC), non-oriented Nylon (CNy), polycarbonate (PC), polystyrene (PS), and hard vinyl chloride (VSC); and a metal foil such as aluminum foil (AL). These materials may be combined, to thereby form a multi-layer structure. Examples of the multi-layer structure include a laminate of PVC and PVDC (hereinafter abbreviated as PVC/PVDC, and the same convention will be employed in the specification), PVC/PVDC/PE/PVC, PVC/PVDC/PE/PVDC/PVC, CPP/COC/CPP, PVC/AL, CPP/AL, CPP/CPP/CPP, or the like. The multi-layer structure may be formed through a known lamination method. Examples of the lamination method include extrusion lamination, dry lamination, co-extrusion lamination, thermal lamination, wet lamination, non-solvent lamination, heat lamination, or the like. The package material of the SP, PTP, pillow package, stick package, and the like is preferably poly(vinyl chloride) or aluminum foil.

In one mode of packing with a PTP, each piece or dosage unit of the solid preparation of the present invention is placed in each of the appropriate numbers of pockets provided through a known method in a resin sheet or the like, and then the pockets are closed with a lid formed of a sheet made of a metal foil (e.g., aluminum foil, or the like). Alternatively, a sheet including aluminum foil as a constituent material may be used for provision of pockets. In this case, a so-called double-sided aluminum PTP package is provided. In the present invention, a PTP is preferably further packed with a pillow package (e.g., an aluminum pillow package, or the like), for enhancing moisture prevention property.

In one mode of packing with an SP, a pillow package, or a stick package, each piece or dosage unit of the solid preparation of the present invention is packed with a package sheet or the like made of a resin sheet or aluminum foil, through a known method. In the present invention, a constituent material of the sheet is preferably aluminum foil, for enhancing moisture prevention property.

The package of the pharmaceutical product of the present invention generally has an occupancy of solid preparation in the package (bulk ratio) of 25 to 90%, preferably 28 to 75%, more preferably 30 to 50%, in the case of a bottle package. In the case of an SP, a PTP, a pillow package, or a stick package, the percent solid preparation content is generally 30 to 98%, preferably 40 to 95%, more preferably 45 to 93%, particularly preferably 50 to 90%. Notably, the "bulk ratio" refers to an occupancy of solid preparation to volume of an internal package, and fillers, an internal stopper, or the like for preventing breakage of the solid preparation stored in the package is not included as the content in calculation of the space occupancy.

In the present invention, the tight package may be a commercial package, or a package prepared by processing a commercial product. Examples of such commercial products include Z series (bottle package products of Hanshin Kasei Kogyo), or the like. Examples of a package material of an SP, a PTP, a pillow package, or a stick package include Sumilite VSS, Sumilite VSL, Sumilite NS, and Sumilite FCL (products of Sumitomo Bakelite Co., Ltd.), TAS series (products of Taiseikako), PTP Vinyfoil and PTP Superfoil (products of Mitsubishi Plastics, Inc.), Nippaku Aluminium Foil (product of UACJ Foil Corporation), Aluminium foil (silvering) (product of Daiwa Chemical Industry Co., Ltd.), or the like.

No particular limitation is imposed on the method of storing the solid preparation in a tight package in the present invention, and the solid preparation can be placed in a package by appropriate means; for example, by feeding the solid preparation into the package. In the above method, a desiccant (e.g., a columnar (tablet) desiccant or a desiccant sheet) may be fed to the package with the solid preparation. However, in order to prevent an excessive time-dependent drop in water content of the solid preparation, the water content of the solid preparation is preferably adjusted in advance to 2.9 mass% or less, without feeding a desiccant.

The solid preparation of the present invention comprises, as an essential ingredient, pitavastatin or a salt thereof having HMG-CoA reductase inhibitory activity, and thus can be used as a therapeutic agent for hyperlipemia, hypercholesterolemia, familial hypercholesterolemia, or the like. The pharmaceutical composition may be administered perorally or parenterally, but peroral administration is preferred. Furthermore, the daily dose of the pharmaceutical composition may be single or divided into about 2 to 4. The composition may be administered, for example, before, during, or after a meal, or before bedtime.

The present invention also discloses the following pharmaceutical products and solid preparations with respect to the aforementioned embodiments.
[1-1] A pharmaceutical product comprising a solid preparation comprising the following ingredients (A) and (B):
   (A) pitavastatin or a salt thereof; and
   (B) at least one member selected from the group consisting of carmellose and a salt thereof, crospovidone, and microcrystalline cellulose,
      and the solid preparation having a water content of 2.9 mass% or less, wherein the solid preparation is stored in a tight package.
[1-2] A pharmaceutical product according to [1-1] above, wherein the solid preparation preferably has a water content of 2.1 mass% or less, more preferably 1.9 mass% or less.
[1-3] A pharmaceutical product according to [1-1] above, wherein the solid preparation preferably has a water content of 1.5 to 2.9 mass%, more preferably 1.5 to 2.1 mass%, even more preferably 1.5 to 1.9 mass%.
[1-4] A pharmaceutical product according to any one of [1-1] to [1-3] above, wherein the solid preparation preferably has an amount of pitavastatin or a salt thereof (ingredient (A)), as reduced to pitavastatin calcium, of 0.1 to 16 mg in a dosage unit, more preferably 0.5 to 12 mg, even more preferably 1 to 8 mg, further more preferably 1 to 4 mg.
[1-5] A pharmaceutical product according to any one of [1-1] to [1-3] above, wherein the solid preparation preferably has an amount of pitavastatin or a salt thereof (ingredient (A)), based on the total mass of the solid preparation and as reduced to pitavastatin calcium, of 0.1 to 10 mass%, more preferably 0.2 to 5 mass%, even more preferably 0.5 to 4 mass%.
[1-6] A pharmaceutical product according to any one of [1-1] to [1-5] above, wherein the solid preparation preferably has a total amount of at least one member selected from the group consisting of carmellose and a salt thereof, crospovidone, and microcrystalline cellulose (ingredient (B)), based on the total mass of the solid preparation, of 0.1 to 85 mass%, more preferably 0.5 to 70 mass%, even more preferably 1 to 60 mass%.
[1-7] A pharmaceutical product according to any one of [1-1] to [1-5] above, wherein the solid preparation preferably has a total amount of at least one member selected from the group consisting of carmellose and a salt thereof, crospovidone, and microcrystalline cellulose (ingredient (B)), with respect to 1 part by mass (as reduced to pitavastatin calcium) of pitavastatin or a salt thereof, of 0.1 to 90 parts by mass, more preferably 0.5 to 75 parts by mass, even more preferably 1 to 65 parts by mass.
[1-8] A pharmaceutical product according to any one of [1-1] to [1-7] above, wherein the solid preparation preferably has a total amount of carmellose and a salt thereof (ingredient (B)), based on the total mass of the solid preparation, of 0.01 to 25 mass%, more preferably 0.1 to 20 mass%, even more preferably 0.5 to 15 mass%.
[1-9] A pharmaceutical product according to any one of [1-1] to [1-7] above, wherein the solid preparation preferably has a total amount of carmellose and a salt thereof (ingredient (B)), with respect to 1 part by mass (as reduced to pitavastatin calcium) of pitavastatin or a salt thereof, of 0.01 to 30 parts by mass, more preferably 0.1 to 25 parts by mass, even more preferably 0.5 to 20 parts by mass.
[1-10] A pharmaceutical product according to any one of [1-1] to [1-9] above, wherein the solid preparation preferably has an amount of crospovidone, based on the total mass of the solid preparation, of 0.1 to 25 mass%, more preferably 0.5 to 20 mass%, even more preferably 1 to 15 mass%.
[1-11] A pharmaceutical product according to any one of [1-1] to [1-9] above, wherein the solid preparation preferably has an amount of crospovidone, with respect to 1 part by mass (as reduced to pitavastatin calcium) of pitavastatin or a salt thereof, of 0.1 to 30 parts by mass, more preferably 0.5 to 25 parts by mass, even more preferably 1 to 20 parts by mass.
[1-12] A pharmaceutical product according to any one of [1-1] to [1-11] above, wherein the solid preparation preferably has an amount of microcrystalline cellulose, based on the total mass of the solid preparation, of 0.1 to 80 mass%, more preferably 0.5 to 65 mass%, even more preferably 1 to 50 mass%.
[1-13] A pharmaceutical product according to any one of [1-1] to [1-11] above, wherein the solid preparation preferably has an amount of microcrystalline cellulose, with respect to 1 part by mass (as reduced to pitavastatin calcium) of pitavastatin or a salt thereof, of 0.1 to 85 parts by mass, more preferably 0.5 to 70 parts by mass, even more preferably 1 to 55 parts by mass.
[1-14] A pharmaceutical product according to any one of [1-1] to [1-13] above, wherein the solid preparation preferably has a pH of 4 or higher, more preferably 4 to 13, even more preferably 5 to 12, even more preferably 6 to 11, further more preferably 7 to 11.
[1-15] A pharmaceutical product according to any one of [1-1] to [1-14] above, wherein the tight package is at least one species selected from the group consisting of a bottle package, an SP, a PTP, a pillow package, and a stick package.
[1-16] A pharmaceutical product according to any one of [1-1] to [1-15] above, wherein the solid preparation is a tablet.
[2-1] A solid preparation comprising the following ingredients (A) and (B):
   (A) pitavastatin or a salt thereof; and
   (B) at least one member selected from the group consisting of carmellose and a salt thereof, crospovidone, and microcrystalline cellulose,
      and the solid preparation having a water content of 2.9 mass% or less.
[2-2] A solid preparation according to [2-1] above, which preferably has a water content of 2.1 mass% or less, more preferably 1.9 mass% or less.
[2-3] A solid preparation according to [2-1] above, which preferably has a water content of 1.5 to 2.9 mass%, more preferably 1.5 to 2.1 mass%, even more preferably 1.5 to 1.9 mass%.
[2-4] A solid preparation according to any one of [2-1] to [2-3] above, which preferably has an amount of pitavastatin or a salt thereof (ingredient (A)), as reduced to pitavastatin calcium, of 0.1 to 16 mg in a dosage unit, more preferably 0.5 to 12 mg, even more preferably 1 to 8 mg, further more preferably 1 to 4 mg.
[2-5] A solid preparation according to any one of [2-1] to [2-3] above, which preferably has an amount of pitavastatin or a salt thereof (ingredient (A)), based on the total mass of the solid preparation and as reduced to pitavastatin calcium, of 0.1 to 10 mass%, more preferably 0.2 to 5 mass%, even more preferably 0.5 to 4 mass%.
[2-6] A solid preparation according to any one of [2-1] to [2-5] above, which preferably has a total amount of at least one member selected from the group consisting of carmellose and a salt thereof, crospovidone, and microcrystalline cellulose (ingredient (B)), based on the total mass of the solid preparation, of 0.1 to 85 mass%, more preferably 0.5 to 70 mass%, even more preferably 1 to 60 mass%.
[2-7] A solid preparation according to any one of [2-1] to [2-5] above, which preferably has a total amount of at least one member selected from the group consisting of carmellose and a salt thereof, crospovidone, and microcrystalline cellulose (ingredient (B)), with respect to 1 part by mass (as reduced to pitavastatin calcium) of pitavastatin or a salt thereof, of 0.1 to 90 parts by mass, more preferably 0.5 to 75 parts by mass, even more preferably 1 to 65 parts by mass.
[2-8] A solid preparation according to any one of [2-1] to [2-7] above, which preferably has a total amount of carmellose and a salt thereof (ingredient (B)), based on the total mass of the solid preparation, of 0.01 to 25 mass%, more preferably 0.1 to 20 mass%, even more preferably 0.5 to 15 mass%.
[2-9] A solid preparation according to any one of [2-1] to [2-7] above, which preferably has a total amount of carmellose and a salt thereof (ingredient (B)), with respect to 1 part by mass (as reduced to pitavastatin calcium) of pitavastatin or a salt thereof, of 0.01 to 30 parts by mass, more preferably 0.1 to 25 parts by mass, even more preferably 0.5 to 20 parts by mass.
[2-10] A solid preparation according to any one of [2-1] to [2-9] above, which preferably has an amount of crospovidone, based on the total mass of the solid preparation, of 0.1 to 25 mass%, more preferably 0.5 to 20 mass%, even more preferably 1 to 15 mass%.
[2-11] A solid preparation according to any one of [2-1] to [2-9] above, which preferably has an amount of crospovidone, with respect to 1 part by mass (as reduced to pitavastatin calcium) of pitavastatin or a salt thereof, of 0.1 to 30 parts by mass, more preferably 0.5 to 25 parts by mass, even more preferably 1 to 20 parts by mass.
[2-12] A solid preparation according to any one of [2-1] to [2-11] above, which preferably has an amount of microcrystalline cellulose, based on the total mass of the solid preparation, of 0.1 to 80 mass%, more preferably 0.5 to 65 mass%, still more preferably 1 to 50 mass%.
[2-13] A solid preparation according to any one of [2-1] to [2-11] above, which preferably has an amount of microcrystalline cellulose, with respect to 1 part by mass (as reduced to pitavastatin calcium) of pitavastatin or a salt thereof, of 0.1 to 85 parts by mass, more preferably 0.5 to 70 parts by mass, even more preferably 1 to 55 parts by mass.
[2-14] A solid preparation according to any one of [2-1] to [2-13] above, which preferably has a pH of 4 or higher, more preferably 4 to 13, even more preferably 5 to 12, even more preferably 6 to 11, further more preferably 7 to 11.
[2-15] A solid preparation according to any one of [2-1] to [2-14] above, which is a tablet.
[3-1] A solid preparation comprising the following ingredients (A) and (B):
   (A) pitavastatin or a salt thereof; and
   (B) at least one member selected from the group consisting of carmellose and a salt thereof, crospovidone,
      and microcrystalline cellulose,
      and the solid preparation having a water content of 1.5 mass% or more.
[3-2] A solid preparation according to [3-1] above, which preferably has a water content of 1.5 to 2.9 mass%, more preferably 1.5 to 2.1 mass%, even more preferably 1.5 to 1.9 mass%.

### Examples

The present invention will next be described in detail by way of examples, which should not be construed as limiting the invention thereto.

### [Test Example 1]

### Checking of production of a lactone form in the co-presence of a disintegrant having high hygroscopicity

Production of a lactone form originating from pitavastatin or a salt thereof in the co-presence of a disintegrant having high hygroscopicity was checked. Specifically, mixtures (the below-described mixtures 1 to 4) were prepared, and each mixture was placed in a polyethylene bag and an aluminum bag and stored at 70°C for 3 days. A lactone form production rate in the mixture was assessed immediately after preparation of the mixture and after 3 day storage at 70°C. The lactone form production rate in each mixture was determined as a ratio (%) of a peak area attributed to a lactone form to the total peak area attributed to pitavastatin and decomposition products thereof. The areas were measured by means of HPLC (model 2695, product of WATERS). As reference, a sample of pitavastatin calcium without coexisting with disintegrant was stored at 70°C for 3 days, and the lactone form production rate was determined in the same manner.

Also, the correlation between production of a lactone form and the water content in the mixture was investigated. Specifically, each of mixtures 1 to 4 was stored at 70°C for 3 days, and the water content thereof was measured.

The water content was determined in accordance with "Water Determination (Karl Fischer Method)" described in "The Japanese Pharmacopoeia (16th edition)," through volumetric titration. Table 1 shows the results.

### <Mixture 1>

Pitavastatin calcium (1 part by mass) was mixed with crospovidone (Kollidon CL-SF, product of BASF) (10 parts by mass), to thereby prepare mixture 1.

### <Mixture 2>

Pitavastatin calcium (1 part by mass) was mixed with carmellose calcium (ECG-505, product of Gotoku Chemical Company Ltd.) (10 parts by mass), to thereby prepare mixture 2.

### <Mixture 3>

Pitavastatin calcium (1 part by mass) was mixed with microcrystalline cellulose (Ceolus UF-711, product of Asahi Kasei Chemicals Corporation) (10 parts by mass), to thereby prepare mixture 3.

### <Mixture 4>

Pitavastatin calcium (1 part by mass) was mixed with carmellose sodium (Cellogen P-815C, product of Dai-Ichi Kogyo Seiyaku Co., Ltd.) (10 parts by mass), to thereby prepare mixture 4.

**[Table 1]**

| Mixture | Type of disintegratnt | Water content (mass%) | Lactone form production rate (%) | |
|---|---|---|---|---|
| | | | Immediately after preparation | 70°C, 3 days |
| 1 | Crospovidone | 5.2 | 0.02 | 0.08 |
| 2 | Carmellose calcium | 5.9 | | 0.16 |
| 3 | Microcrystalline cellulose | 10.1 | | 0.33 |
| 4 | Carmellose sodium | 11.7 | | 0.32 |

| | | | | |
|---|---|---|---|---|
| *Lactone form production rate (70°C, 3 days) of a sample of pitavastatin calcium without coexisting with disintegrant was 0.03% | | | | |

As is clear from the test results shown in Table 1, the water content of each mixture was found to correlate with lactone form production rate. Specifically, as the water content increased, the amount of produced lactone form increased. Since crospovidone, carmellose calcium, microcrystalline cellulose, and carmellose sodium have high hygroscopicity, production of a lactone form is conceivably attributed to high hygroscopicity of such a disintegrant. Conceivably, the co-present disintegrant absorbs water, and pitavastatin comes in contact with water, whereby a lactone form is produced.

### [Test Example 2]

### Lactone form production suppression test

Several types of orally disintegrating tablets were produced through the below-described method. The water content of each tablet was adjusted through vacuum drying to a level shown in Table 3 (Water content measured through the below-described method). Subsequently, each of the tablets (100 pieces) having a controlled water content was placed in a pocket provided in a resin sheet (Sumilite VSS-1202-R, product of Sumitomo Bakelite Co., Ltd.), and then the pocket was closed with a lid formed of PTP Aluminium foil (plain silver) (product of Daiwa Chemical Industry Co., Ltd.), to thereby complete PTP. The thus-obtained PTP was further packed with an aluminum laminate bag (Lamizip AL series, product of Seisannipponsha Ltd.), to thereby complete aluminum pillow packing. The thus-obtained package was stored at 40°C and 75% RH for 2 months. Lactone form production rate of the tablet was assessed immediately after tablet preparation and 1 and 2 months after start of storage. Lactone form production rate in each tablet was determined as a ratio (%) of a peak area attributed to a lactone form to the total peak area attributed to pitavastatin and decomposition products thereof. The areas were measured by means of HPLC (model LC-20 series, product of SHIMADZU).

Water content of the tablet was determined in accordance with "Water Determination (Karl Fischer Method)" described in "The Japanese Pharmacopoeia (16th edition)," through volumetric titration.

### <Production of orally disintegrating tablets>

Orally disintegrating tablets containing the ingredients in amounts per tablet (mg) shown in Table 2 below were produced through a routine method.

Specifically, ingredients (from pitavastatin calcium to titanium oxide in Table 2) were wet-granulated, to form granules.

The thus-obtained granules were mixed with the other ingredients (from xylitol in Table 2), and the resultant mixture was pelletized, to thereby produce orally disintegrating tablets (120 mg/tablet).

**[Table 2]**

| | Formulation 1 |
|---|---|
| Pitavastatin calcium | 1.0 |
| Crospovidone | 3.0 |
| D-Mannitol | 20.0 |
| Hypromellose | 2.6 |
| Sucralose | 1.0 |
| I-Menthol | 0.3 |
| Calcium chloride dihydrate | 1.0 |
| Magnesium aluminometasilicate | 0.6 |
| Yellow ferric oxide | 0.7 |
| Titanium oxide | 5.0 |

| The above ingredients forming granules | |
|---|---|
| Xylitol | 3.0 |
| D-Mannitol | 23.0 |
| Microcrystalline cellulose | 45.0 |
| Crospovidone | 10.6 |
| Aminoalkyl methacrylate copolymer E | 0.5 |
| Anhydrous dibasic calcium phosphate | 2.0 |
| Yogurt micron | 0.1 |
| Calcium stearate | 0.6 |
| Total | 120.0 |

Table 3 shows the results.

**[Table 3]**

| Water content (mass%) | Lactone form production rate (%) | | |
|---|---|---|---|
| | Immediatel y after preparation | 40°C, 75% RH, 1 month | 40°C, 75% RH, 2 months |
| 1.5 | 0.05 | 0.09 | 0.12 |
| 1.9 | 0.05 | 0.10 | 0.13 |
| 2.1 | 0.05 | 0.11 | 0.14 |
| 2.9 | 0.05 | 0.15 | 0.23 |
| 3.3 | 0.04 | 0.23 | 0.35 |

As is clear from the test results shown in Table 3, when the tablet had a water content of 2.9 mass% or less, production of a lactone form was remarkably suppressed. Particularly when the tablet had a water content of 2.1 mass% or less, lactone form production rate was maintained at a low level even after storage at 40°C and 75% RH for 2 months. Notably, entry of water into the package was impeded by means of a tight package (including PTP and an aluminum pillow package). As a result, there was no change in the water content of each tablet even after storage at 40°C and 75% RH for 2 months.

The above test has revealed that production of a lactone form is suppressed in a solid preparation comprising pitavastatin or a salt thereof, and at least one member selected from the group consisting of carmellose and a salt thereof, crospovidone, and microcrystalline cellulose, and having a water content of 2.9 mass% or less. Also, a pharmaceutical product comprising the solid preparation which is stored in a tight package has a stably-maintained water content, whereby production of a lactone form can be suppressed for a long period of time.

### [Test Example 3]

### 5-Keto form production suppression test

Through the same procedure as employed in Test Example 2, orally disintegrating tablets having different water contents were packed with a PTP and then with an aluminum pillow package. The thus-obtained packages were stored at 40°C and 75% RH for 2 months. 5-keto form production rate of each tablet was assessed immediately after tablet preparation and 1 and 2 months after start of storage. 5-keto form production rate in each tablet was determined as a ratio (%) of a peak area attributed to a 5-keto form to the total peak area attributed to pitavastatin and decomposition products thereof. The areas were measured by means of HPLC (model LC-20 series, product of SHIMADZU).

Table 4 shows the results.

**[Table 4]**

| Water content (mass%) | 5-keto form production rate (%) | | |
|---|---|---|---|
| | Immediately after preparation | 40°C, 75% RH, 1 month | 40°C, 75% RH, 2 months |
| 1.5 | 0.13 | 0.16 | 0.18 |
| 1.9 | 0.14 | 0.16 | 0.18 |
| 2.1 | 0.14 | 0.17 | 0.17 |
| 2.9 | 0.14 | 0.14 | 0.14 |

As is clear from the test results shown in Table 4, when the tablet had a water content of 1.5 mass% or more, production of a 5-keto form was remarkably suppressed. Notably, entry of water into or out of the package was impeded by means of a tight package (including PTP and an aluminum pillow package). As a result, there was no change in the water content of each tablet even after storage at 40°C and 75% RH for 2 months.

The above test has revealed that production of a 5-keto form is suppressed in a solid preparation comprising pitavastatin or a salt thereof, and at least one member selected from the group consisting of carmellose and a salt thereof, crospovidone, and microcrystalline cellulose, and having a water content of 1.5 mass% or more.

### Production Example 1

Through the same procedure as employed in Test Example 2, orally disintegrating tablets of Formulation Example 2 (F2) containing the ingredients in amounts per tablet (mg) shown in Table 5 below are produced. The tablets are dried by means of a box-type drier so as to adjust the water content to about 2.5 mass% and then placed in a bottle made of high-density polyethylene, to thereby yield a pharmaceutical product of Production Example 1.

### Production Example 2

Through the same procedure as employed in Test Example 2, orally disintegrating tablets of Formulation Example 3 (F3) containing the ingredients in amounts per tablet (mg) shown in Table 5 below are produced. The tablets are dried by means of a fluidized-bed drier so as to adjust the water content to about 2.4 mass%. Each tablet is placed in each of the pockets provided in a resin sheet (Sumilite VSS-1202, product of Sumitomo Bakelite Co., Ltd.), and then the pockets are closed with a lid formed of PTP Aluminium foil (plain silver) (product of Daiwa Chemical Industry Co., Ltd.), to thereby complete PTP. Three sheets of the thus-prepared PTP (10 orally disintegrating tablets are stored per sheet) are packed with an aluminum pillow package, to thereby yield a pharmaceutical product of Production Example 2.

### Production Example 3

Through the same procedure as employed in Test Example 2, orally disintegrating tablets of Formulation Example 4 (F4) containing the ingredients in amounts per tablet (mg) shown in Table 5 below are produced. The tablets are dried through co-presence with a desiccant (silica gel) for one day so as to adjust the water content to about 2.3 mass%. Each tablet is placed in each of the pockets provided in a resin sheet (Sumilite VSS-1104, product of Sumitomo Bakelite Co., Ltd.), and then the pockets is closed with a lid formed of PTP Aluminium foil (plain silver) (product of Daiwa Chemical Industry Co., Ltd.), to thereby complete PTP. Two sheets of the thus-prepared PTP (12 orally disintegrating tablets are stored per sheet) are packed with an aluminum pillow package, to thereby yield a pharmaceutical product of Production Example 3.

### Production Example 4

Through the same procedure as employed in Test Example 2, orally disintegrating tablets of Formulation Example 5 (F5) containing the ingredients in amounts per tablet (mg) shown in Table 5 below are produced. The tablets are dried by means of a box-type drier so as to adjust the water content to about 1.8 mass%. Each tablet is placed in each of the pockets provided in a resin sheet (Sumilite VSL-4501, product of Sumitomo Bakelite Co., Ltd.), and then the pockets are closed with a lid formed of PTP Aluminium foil (plain silver) (product of Daiwa Chemical Industry Co., Ltd.), to thereby complete PTP. Three sheets of the thus-prepared PTP (10 orally disintegrating tablets are stored per sheet) are packed with an aluminum pillow package, to thereby yield a pharmaceutical product of Production Example 4.

### Production Example 5

Through the same procedure as employed in Test Example 2, orally disintegrating tablets of Formulation Example 6 (F6) containing the ingredients in amounts per tablet (mg) shown in Table 5 below are produced. The tablets are dried by means of a high-frequency drier so as to adjust the water content to about 1.6 mass%. The tablets are placed in a glass bottle, to thereby yield a pharmaceutical product of Production Example 5.

### Production Example 6

Through the same procedure as employed in Test Example 2, orally disintegrating tablets of Formulation Example 7 (F7) containing the ingredients in amounts per tablet (mg) shown in Table 5 below are produced. The tablets are dried through co-presence with a desiccant (synthetic zeolite) for one day so as to adjust the water content to about 2.2 mass%. The tablets are packed with SP aluminum foil (product of Nissan Kako Co., Ltd.), to thereby yield a pharmaceutical product of Production Example 6.

**[Table 5]**

| | F2 | F3 | F4 | F5 | F6 | F7 |
|---|---|---|---|---|---|---|
| D-Mannitol | 16.6 | - | 14.6 | - | 14.6 | - |
| Cornstarch | - | 14.6 | - | 14.6 | - | 14.6 |
| Crospovidone | 5.6 | - | - | - | - | - |
| Microcrystalline cellulose | - | 5.6 | - | - | - | - |
| Croscarmellose sodium | - | - | 5.6 | - | - | - |
| Carmellose calcium | - | - | - | 5.6 | - | - |
| Carmellose sodium | - | - | - | - | 5.6 | - |
| Carmellose | - | - | - | - | - | 5.6 |
| Pitavastatin calcium | 2.0 | 2.0 | 1.0 | 2.0 | 2.0 | 4.0 |
| Hypromellose | 4.4 | 4.4 | 4.4 | 4.4 | 4.4 | 4.4 |
| Sucralose | 2.0 | 2.0 | 1.0 | 2.0 | 2.0 | 2.0 |
| I-Menthol | 0.4 | 0.4 | - | 0.4 | 0.4 | 0.4 |
| Calcium chloride dihydrate | 1.0 | - | - | 1.0 | 2.0 | - |
| Magnesium chloride hexahydrate | - | - | - | 1.0 | - | - |
| Magnesium aluminometasilicate | 1.2 | - | 5.0 | 1.2 | 1.2 | - |
| Precipitated calcium carbonate | - | 3.0 | - | - | - | 1.2 |
| Magnesium oxide | 1.0 | - | 5.0 | - | 2.0 | 2.0 |
| Aminoalkyl methacrylate copolymer E | - | - | - | 3.0 | 3.0 | - |
| Yellow ferric oxide | 1.0 | 0.6 | 1.0 | 1.0 | 1.0 | 1.0 |
| Titanium oxide | 7.4 | 7.4 | 7.4 | 7.4 | 7.4 | 7.4 |

| The above ingredients forming granules | | | | | | |
|---|---|---|---|---|---|---|
| D-Mannitol | 40.0 | 42.6 | 43.6 | 42.0 | 40.0 | 40.0 |
| Xylitol | 3.0 | 3.0 | 3.0 | 3.0 | 3.0 | 3.0 |
| Crospovidone | - | - | - | 12.9 | - | - |
| Microcrystalline cellulose | 55.0 | 55.0 | 50.0 | - | - | - |
| Croscarmellose sodium | 12.9 | - | - | - | 12.9 | - |
| Carmellose calcium | - | 12.9 | - | - | - | 12.9 |
| Carmellose sodium | - | - | 12.9 | - | - | - |
| Carmellose | - | - | - | 55.0 | 55.0 | 55.0 |
| Anhydrous dibasic calcium phosphate | 2.5 | 2.5 | 2.5 | 2.5 | 2.5 | 2.5 |
| Aminoalkyl methacrylate copolymer E | 3.0 | 3.0 | 2.0 | - | - | 3.0 |
| Yogurt micron | 0.2 | - | - | 0.2 | - | - |
| Orange micron | - | 0.2 | 0.2 | - | 0.2 | 0.2 |
| Calcium stearate | 0.8 | - | 0.8 | 0.8 | 0.8 | - |
| Magnesium stearate | - | 0.8 | - | - | - | 0.8 |
| Total | 160.0 | 160.0 | 160.0 | 160.0 | 160.0 | 160.0 |

### Production Example 7

Through a conventional method, tablets of Formulation Example 8 (F8) containing the ingredients in amounts per tablet (mg) shown in Table 6 below are produced. The tablets are dried by means of a fluidized-bed drier so as to adjust the water content to about 2.3 mass%. Each tablet is placed in each of the pockets provided in a resin sheet (Sumilite VSS-1202, product of Sumitomo Bakelite Co., Ltd.), and then the pockets are closed with a lid formed of PTP Aluminium foil (plain silver) (product of Daiwa Chemical Industry Co., Ltd.), to thereby complete PTP. Three sheets of the thus-prepared PTP (10 tablets are stored per sheet) are packed with an aluminum pillow package, to thereby yield a pharmaceutical product of Production Example 7.

### Production Example 8

Through a conventional method, tablets of Formulation Example 9 (F9) containing the ingredients in amounts per tablet (mg) shown in Table 6 below are produced. The tablets are dried through co-presence with a desiccant (silica gel) for one day so as to adjust the water content to about 1.8 mass%. Each tablet is placed in each of the pockets provided in a resin sheet (Sumilite VSS-1104, product of Sumitomo Bakelite Co., Ltd.), and then the pockets are closed with a lid formed of PTP Aluminium foil (silvering) (product of Daiwa Chemical Industry Co., Ltd.), to thereby complete PTP. Two sheets of the thus-prepared PTP (12 tablets are stored per sheet) are packed with an aluminum pillow package, to thereby yield a pharmaceutical product of Production Example 8.

### Production Example 9

Through a conventional method, tablets of Formulation Example 10 (F10) containing the ingredients in amounts per tablet (mg) shown in Table 6 below were produced. The tablets are dried by means of a box-type drier so as to adjust the water content to about 1.5 mass%. Each tablet is placed in each of the pockets provided in a resin sheet (Sumilite VSL-4501, product of Sumitomo Bakelite Co., Ltd.), and then the pockets are closed with a lid formed of PTP Aluminium foil (plain silver) (product of Daiwa Chemical Industry Co., Ltd.), to thereby complete PTP. Three sheets of the thus-prepared PTP (10 tablets are stored per sheet) are packed with an aluminum pillow package, to thereby yield a pharmaceutical product of Production Example 9.

### Production Example 10

Through a conventional method, tablets of Formulation Example 11 (F11) containing the ingredients in amounts per tablet (mg) shown in Table 6 below are produced. The tablets are dried by means of a high-frequency drier so as to adjust the water content to about 2.6 mass%. The tablets are placed in a glass bottle, to thereby yield a pharmaceutical product of Production Example 10.

### Production Example 11

Through a conventional method, tablets of Formulation Example 12 (F12) containing the ingredients in amounts per tablet (mg) shown in Table 6 below are produced. The tablets are dried through co-presence with a desiccant (synthetic zeolite) for one day so as to adjust the water content to about 2.1 mass%. The tablets are packed with SP aluminum foil (product of Nissan Kako Co., Ltd.), to thereby yield a pharmaceutical product of Production Example 11.

### Production Example 12

Through a conventional method, tablets of Formulation Example 13 (F13) containing the ingredients in amounts per tablet (mg) shown in Table 6 below are produced. The tablets are dried by means of a box-type drier so as to adjust the water content to about 1.9 mass% and then placed in a bottle made of low-density polyethylene, to thereby yield a pharmaceutical product of Production Example 12.

**[Table 6]**

| | F8 | F9 | F10 | F11 | F12 | F13 |
|---|---|---|---|---|---|---|
| Pitavastatin calcium | 1.0 | 2.0 | 1.0 | 2.0 | 4.0 | 1.0 |
| Crospovidone | 12.0 | - | - | - | - | - |
| Microcrystalline cellulose | - | 12.0 | - | - | - | - |
| Croscarmellose sodium | - | - | 12.0 | - | - | - |
| Carmellose calcium | - | - | - | 12.0 | - | - |
| Carmellose sodium | - | - | - | - | 12.0 | - |
| Carmellose | - | - | - | - | - | 12.0 |
| Lactose | 98.6 | 97.8 | 92.8 | 97.6 | 93.6 | 98.6 |
| Hypromellose | 2.0 | 2.0 | 2.0 | 2.0 | 2.0 | 2.0 |
| Sucralose | 2.0 | 2.0 | 1.0 | 2.0 | 2.0 | 2.0 |
| Calcium chloride dihydrate | 1.0 | - | - | 1.0 | 2.0 | - |
| Magnesium chloride hexahydrate | - | - | - | 1.0 | - | - |
| Magnesium aluminometasilicate | 1.2 | - | 5.0 | 1.2 | 1.2 | - |
| Precipitated calcium carbonate | - | 3.0 | - | - | - | 1.2 |
| Magnesium oxide | 1.0 | - | 5.0 | - | 2.0 | 2.0 |
| Calcium stearate | 1.2 | - | 1.2 | 1.2 | 1.2 | - |
| Magnesium stearate | - | 1.2 | - | - | - | 1.2 |
| Total | 120.0 | 120.0 | 120.0 | 120.0 | 120.0 | 120.0 |

### Production Example 13

Through a conventional method, tablets of Formulation Example 14 (F14) containing the ingredients in amounts per tablet (mg) shown in Table 7 below are produced. The tablets are dried by means of a high-frequency drier so as to adjust the water content to about 2.7 mass%. The tablets are placed in a glass bottle, to thereby yield a pharmaceutical product of Production Example 13.

### Production Example 14

Through a conventional method, tablets of Formulation Example 15 (F15) containing the ingredients in amounts per tablet (mg) shown in Table 7 below are produced. The tablets are dried through co-presence with a desiccant (synthetic zeolite) for one day so as to adjust the water content to about 2.5 mass%. The tablets are packed with SP aluminum foil (product of Nissan Kako Co., Ltd.), to thereby yield a pharmaceutical product of Production Example 14.

### Production Example 15

Through a conventional method, tablets of Formulation Example 16 (F16) containing the ingredients in amounts per tablet (mg) shown in Table 7 below are produced. The tablets are dried by means of a box-type drier so as to adjust the water content to about 2.0 mass% and then placed in a bottle made of low-density polyethylene, to thereby yield a pharmaceutical product of Production Example 15.

**[Table 7]**

| | F14 | F15 | F16 |
|---|---|---|---|
| Pitavastatin calcium | 8.0 | 8.0 | 8.0 |
| Microcrystalline cellulose | 30.0 | - | - |
| Carmellose calcium | - | 30.0 | - |
| Carmellose sodium | - | - | 30.0 |
| Lactose | 73.8 | 73.6 | 71.6 |
| Hypromellose | 2.0 | 2.0 | 2.0 |
| Sucralose | 2.0 | 2.0 | 2.0 |
| Calcium chloride dihydrate | - | 1.0 | 2.0 |
| Magnesium chloride hexahydrate | - | 1.0 | - |
| Magnesium aluminometasilicate | - | 1.2 | 1.2 |
| Precipitated calcium carbonate | 3.0 | - | - |
| Magnesium oxide | - | - | 2.0 |
| Calcium stearate | - | 1.2 | 1.2 |
| Magnesium stearate | 1.2 | - | - |
| Total | 120.0 | 120.0 | 120.0 |

### Industrial Applicability

According to the present invention, production of a lactone form of pitavastatin or a salt thereof is suppressed, to thereby provide pharmaceuticals having high stability of active ingredients. Such pharmaceuticals can be employed in the pharmaceutical field or the like.

## Claims

1. A pharmaceutical product comprising a solid preparation comprising the following ingredients (A) and (B):
(A) pitavastatin or a salt thereof; and
(B) at least one member selected from the group consisting of carmellose and a salt thereof, crospovidone, and microcrystalline cellulose,
and the solid preparation having a water content of 2.9 mass% or less, wherein the solid preparation is stored in a tight package.

2. A pharmaceutical product according to claim 1,
wherein the solid preparation has a water content of 1.5 to 2.9 mass%.

3. A pharmaceutical product according to claim 1 or 2,
wherein the tight package is at least one member selected from the group consisting of a bottle package, an SP, a PTP, a pillow package, and a stick package.

4. A pharmaceutical product according to any one of claims 1 to 3, which comprises pitavastatin or a salt thereof in an amount, as reduced to pitavastatin calcium, of 0.1 to 16 mg in a dosage unit of the solid preparation.

5. A pharmaceutical product according to any one of claims 1 to 4, wherein the solid preparation is a tablet.

6. A solid preparation comprising the following ingredients (A) and (B):
(A) pitavastatin or a salt thereof; and
(B) at least one member selected from the group consisting of carmellose and a salt thereof, crospovidone, and microcrystalline cellulose,
and the solid preparation having a water content of 2.9 mass% or less.

7. A solid preparation according to claim 6, which has a water content of 1.5 to 2.9 mass%.

8. A solid preparation according to claim 6 or 7, which comprises pitavastatin or a salt thereof in an amount, as reduced to pitavastatin calcium, of 0.1 to 16 mg in a dosage unit of the solid preparation.

9. A solid preparation according to any one of claims 6 to 8, wherein the solid preparation is a tablet.
